(19) Europäisches Patentamt
European Patent Office
Office européen des brevets

(11) Veröffentlichungsnummer: **0 037 481**
**B1**

(12) # EUROPÄISCHE PATENTSCHRIFT

(45) Veröffentlichungstag der Patentschrift: **29.06.83**

(51) Int. Cl.³: **C 07 C 125/03**

(21) Anmeldenummer: **81101902.5**

(22) Anmeldetag: **14.03.81**

(54) **Verfahren zur Herstellung von 1-Monochloräthylcarbamidsäurechlorid.**

(30) Priorität: **05.04.80 DE 3013270**

(43) Veröffentlichungstag der Anmeldung:
**14.10.81 Patentblatt 81/41**

(45) Bekanntmachung des Hinweises auf die Patenterteilung:
**29.06.83 Patentblatt 83/26**

(84) Benannte Vertragsstaaten:
**AT BE CH DE FR GB IT LI NL**

(56) Entgegenhaltungen:
**DE-B-1 154 087**

(73) Patentinhaber: **BASF Aktiengesellschaft,
Carl-Bosch-Strasse 38, D-6700 Ludwigshafen (DE)**

(72) Erfinder: **Koenig, Karl-Heinz, Dr., Pierstrasse 8 A,
D-6710 Frankenthal (DE)**
Erfinder: **Feuerherd, Karl-Heinz, Dr., Berner Weg 34,
D-6700 Ludwigshafen (DE)**

**EP 0 037 481 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 1-Monochloräthylcarbamidsäurechlorid

Die Erfindung betrifft ein Verfahren zur Herstellung von 1-Monochloräthylcarbamidsäurechlorid durch Chlorierung von Äthylisocyanat oder Äthylcarbamidsäurechlorid bei −78 bis 0 °C unter Belichtung.

Die Synthese von 1-Monochloräthylcarbamidsäurechlorid wurde bisher meist nach dem in DE-OS 27 41 980 beschriebenen Verfahren durch Umsetzung von N-Vinyl-N-tert.-butylcarbamidsäurechlorid und nach dem in DE-OS 27 32 284 beschriebenen Verfahren durch Umsetzung von Vinylisocyanat mit Chlorwasserstoff durchgeführt. Diese Verfahren sind wirtschaftlich aufwendig, da die Ausgangsstoffe schwer zugänglich sind.

Es ist aus der DE-OS 14 18 666 bekannt, dass sich 2-Chloräthylisocyanat in Gegenwart von Chlor unter UV-Belichtung zu 21% in Dichloräthylisocyanat und zu 37,2% in Trichloräthylisocyanat umwandelt (Beispiel 2). Verwendet man Sulfurylchlorid als Chlorspender, so erhält man in 94% Ausbeute das Trichloräthylisocyanat, das nur ca. 10% Carbamidsäurechlorid enthält. Als Carbamidsäurechlorid bildet sich nur das Trichloräthylcarbamidsäurechlorid.

In der DE-PS 11 22 058 wird ein Verfahren zur Herstellung von 1-Halogenalkylisocyanaten auf Basis von Alkylisocyanaten oder Alkylcarbamidsäurehalogeniden durch Umsetzung mit Halogenierungsmitteln beschrieben. Dabei entstehen jedoch beträchtliche Anteile von Polymeren. In allen Beispielen (2, 6) wird kein Äthylisocyanat, sondern stets β-Chloräthylisocyanat verwendet, wobei die Chlorierung bei 80 bis 150 °C unter UV-Licht Gemische von β-Chlor-, α,β-Dichlor-, β,β-Dichlor-, isomeren Trichlor-, isomeren Tetrachlor- sowie Pentachlor-äthylisocyanaten ergibt. Es wird betont, dass die Umsetzung von Alkylisocyanaten mit Halogenierungsmitteln glatt zu den halogenierten Isocyanaten führt. Die Bildung von halogenierten Carbamidsäurechloriden wird nicht erwähnt. Es wird darauf aufmerksam gemacht, dass mindestens bei Zimmertemperatur, zweckmässig auch bei erhöhter Temperatur oder Belichtung oder unter Verwendung von Halogenüberträgern wie Eisen-III-chlorid, umgesetzt werden soll.

Es ist aus der Angewandten Chemie, Band 74 (1962), Seiten 848 bis 855 bekannt, dass man Alkylcarbamidsäurechloride mit elementarem Chlor in die entsprechenden α-Chloralkylcarbamidsäurechloride umsetzt. Die dabei anfallenden Produkte sind jedoch Gemische sowohl mit Bezug auf den Halogenierungsgrad wie auf die Stellung der eintretenden Halogenatome. Das Verfahren ist im Hinblick auf Ausbeute und Reinheit des Endstoffs sowie einfachen und wirtschaftlichen Betrieb nicht befriedigend. Das genannte Verfahren wird als so ungünstig betrachtet, dass die deutsche Offenlegungsschrift 27 32 284 von einer direkten Chlorierung abgeht und die vorgenannte Umsetzung mit Halogenwasserstoff empfiehlt, weil nur so auf einfacherem und wirtschaftlicherem Wege α-Halogenäthylcarbamidsäurehalogenide in besserer Ausbeute und Reinheit und eine einfachere Aufarbeitung, da kein komponentenreiches Reaktionsgemisch erhalten wird, zu erzielen sei.

Auch Synthesis (1980), Seiten 85 bis 110, lehren ausdrücklich (Seite 90), dass die Chlorierung von Alkylisocyanaten unter Erhitzen, z.B. bei 55 bis 60 °C, und UV-Bestrahlung zu α-Chloralkylisocyanaten führt. Eine Bildung von entsprechenden Carbamidsäurechloriden wird nicht erwähnt. Wie die Beispiele der Tabelle 7 zeigen, werden im Falle von Alkylisocyanaten höherchlorierte, perchlorierte Isocyanate oder entsprechende Gemische erzielt. Während sowohl Methylisocyanat wie auch Benzylisocyanat ohne Halogensubstituenten verwendet werden, werden auch hier keine halogenfreien und auch keinen α-halogenierten Ethylisocyanate, sondern stets β-halogenierte und zwar β-perhalogenierte Ethylisocyanate verwendet.

Die DE-B-1 154 087 beschreibt ein Verfahren zur Herstellung von halogenierten N,N-disubstituierten Carbamidsäurehalogeniden durch Umsetzung von N,N-Dialkylcarbamidsäurehalogeniden mit Halogenierungsmitteln. Die Ausgangsverbindungen enthalten keine N-H-Funktionen; somit können bei der Reaktion mit Chlor oder anderen Halogenierungsmitteln keine zu Nebenreaktionen führenden N-Chlorverbindungen entstehen. Die im Reaktionsgemisch vorliegenden Endstoffe können nicht in einer chlorotropen Form vorliegen. In den Beispielen werden Gemische von mehrfach halogenierten Verbindungen erhalten, die durch fraktionierte Destillation aufwendig getrennt werden müssen. Wie Beispiel 1 zeigt, sind nicht halogenierter Ausgangsstoff, einfach, zweifach und mehrfach halogenierte Endstoffe im Endprodukt der Reaktion in erheblichen Mengen vorhanden.

Es wurde nun gefunden, dass man 1-Monochloräthylcarbamidsäurechoorid durch Umsetzung von Isocyanaten oder Äthylcarbamidsäurechlorid mit Halogenierungsmitteln vorteilhaft erhält, wenn man Äthylisocyanat oder Äthylcarbamidsäurechlorid mit Chlor bei einer Temperatur von −78 bis 0 °C unter Belichtung umsetzt.

Weiterhin wurde gefunden, dass man das Verfahren vorteilhaft anwendet, wenn während der Reaktion ein Überschuss an freiem Chlor von höchstens 0,1 Mol je Mol Äthylisocyanat verwendet wird.

Die Umsetzung lässt sich durch die folgenden Formeln wiedergeben:

$$CH_3\text{–}CH_2\text{–}N{=}C{=}O + Cl_2 \rightarrow CH_3\text{–}\underset{\displaystyle Cl}{\underset{|}{C}}H\text{–}NH\text{–}\overset{\displaystyle O}{\overset{\|}{C}}\text{–}Cl$$

Im Hinblick auf den Stand der Technik liefert das Verfahren nach der Erfindung überraschend auf einfacherem und wirtschaftlicherem Wege selektiv 1-Monochloräthylcarbamidsäurechlorid in hoher Ausbeute und Reinheit. Schwerer zugängliche Halogenierungsmittel, wie Sulfurylchlorid, sind nicht notwendig. Sowohl das Halogenierungsmittel Chlor wie auch das verwendete Äthylisocyanat sind leicht zugänglich. Aufwendige Reinigungs- und Aufarbeitungsoperationen werden eingespart, da keine heterogenen Gemische von Komponenten verschiedenen Halogenierungsgrades anfallen. Alle diese vorteilhaften Ergebnisse des erfindungsgemässen Verfahrens sind überraschend. Im Hinblick auf den Stand der Technik hätte man bei den erfindungsgemässen Bedingungen Isocyanate und keine Carbamidsäurechloride als Endstoffe erwarten müssen. Auch war es überraschend, dass chloriertes und noch dazu in 1-Stellung monochloriertes Äthylcarbamidsäurechlorid als hauptsächlicher Endstoff in so hoher Reinheit anfällt. Auch zeigt die deutsche Patentschrift 11 22 058 (Beispiel 1), dass ja bei Chlorierung unter Belichtung bei 40 bis 70 °C gerade aus Methylcarbamidsäurechlorid Chlormethylisocyanat hergestellt wird, die umgekehrte Reaktion somit nicht stattfindet. In der Vorlage bildet sich Chlormethylcarbamidsäurechlorid, aber aus dem schon chlorierten Chlormethylisocyanat und Chlorwasserstoff.

Insbesondere musste bei den erfindungsgemässen Bedingungen neben der hauptsächlichen Bildung von Isocyanaten die Bildung heterogener Gemische erwartet werden. Im Hinblick auf Beispiel 6 der deutschen Patentschrift 11 22 058 musste mit Gemischen von 2 Monochloräthylisocyanaten, 3 Dichloräthylisocyanaten, 3-Trichloräthylisocyanaten, 2-Tetrachloräthylisocyanaten und einem Pentachloräthylisocyanat gerechnet werden, über deren Mengenverhältnis aber gerade Beispiel 6 keinen Hinweis geben kann, da hier schon eine gewisse Richtung der Umsetzung durch die Verwendung von β-Chloräthylisocyanat gegeben war. Konnte somit auch bei detaillierter Betrachtung höchstens eine geringfügige Bildung von Carbamidsäurechloriden spekulativ vermutet werden, so mussten auf jeden Fall dann weitere 11 unterschiedliche Carbamidsäurechloridkomponenten als Ergebnis der Umsetzung erwartet werden.

Auch musste es fraglich erscheinen, ob α-Monochloräthylisocyanat überhaupt in deutlicher Weise durch Chlorierung aus Äthylisocyanat zu erhalten wäre, da der Stand der Technik kein Beispiel zeigt und stets nur β-substituierte Äthylisocyanate, z.B. Trichlor- oder β-Chlorverbindungen verwendet. Aber auch in diesen Fällen erhält man aus einem β-Monochlorethylisocyanat nur Gemische und kein reines α,β-Dichlorethylisocyanat. Insgesamt musste die Bildung von reinem α,β-Dichlorethylisocyanat und noch weit mehr von α-Monochlorethylcarbamidsäurechlorid somit unter den erfindungsgemässen Bedingungen ausgeschlossen werden.

Im Vergleich zu dem in DE-B-1 154 087 beschriebenen Verfahren weist das erfindungsgemässe Verfahren wesentliche Unterschiede auf. Eine Reaktion von Chlor mit der N-H-Funktion war zu erwarten. Die erfindungsgemässen Endstoffe liegen nach der Lehre von Angewandte Chemie, Band 74 (1962), Seite 848, Spalte 2, letzter Absatz, in einem chlorotropen Gleichgewicht vor

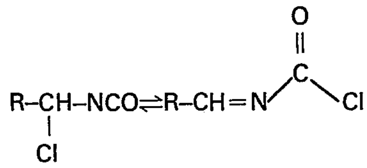

und eine Addition von Chlor an die CN-Doppelbindung war daher zu vermuten. Beim erfindungsgemässen Verfahren entsteht in selektiver Reaktion und bei hohem Umsatz nur die monohalogenierte Verbindung. Man hätte im Hinblick auf diese Druckschrift bei dem erfindungsgemässen Verfahren als Endstoff ein Gemisch heterogener Chlorverbindungen, die in der Ethylgruppe einfach, zweifach und mehrfach halogeniert sind, N-Chlorverbindungen und ihrer entsprechenden Zersetzungsprodukte erwartet. Die erfindungsgemässe einfache und selektive Umsetzung zur 1-Monochlorethyl-Verbindung und die erfindungsgemässen Ausbeuten an Endstoff waren daher überraschend.

Die Herstellung des Ausgangsstoffs kann z.B. nach den in Ullmanns Encyklopädie der technischen Chemie, Band 13 (4. Auflage, 1977), Seiten 350 bis 354, beschriebenen Verfahren durchgeführt werden. Man verwendet das Chlor in stöchiometrischer Menge, im Unterschuss oder im Überschuss, vorzugsweise in einer Menge von 0,1 bis 1,5, insbesondere 0,5 bis 1,1 Mol Chlor je Mol Äthylisocyanat. Die Umsetzung wird bei einer Temperatur von −78 °C bis 0 °C, zweckmässig zwischen −75 und 0 °C, insbesondere bei −40 bis −10 °C, drucklos oder unter Druck, vorzugsweise bei 0,3 bis 5, insbesondere 0,7 bis 2 bar, kontinuierlich oder diskontinuierlich durchgeführt. Man kann ohne Lösungsmittel umsetzen, zweckmässig verwendet man aber in Bezug auf Äthylisocyanat unter den Reaktionsbedingungen inerte Lösungsmittel, die aufgrund ihres tieferen Schmelzpunktes für die gewählte Chlorierungstemperatur geeignet sind. Wasser wird nicht verwendet. Bevorzugt arbeitet man in Lösungsmitteln, die bei der weiteren Umsetzung des Endstoffs als Reaktionsmedien Verwendung finden. Als Lösungsmittel kommen z.B. in Frage (Fp. in Klammern): Halogenkohlenwasserstoffe wie Dichlormethan (−96 °C), Trichlormethan (−63,5 °C), Tetrachlormethan (−22,9 °C), Trichloräthylen (−83 °C), tert.-Butylchlorid (−28,5 °C), 1,2-Dichloräthan (−35,5 °C), 1,2-Dibrompropan (−55 °C), 1,2-Dichlorpropan (−100 °C), 1,4-Dibrombutan (−20 °C), Chlorbenzol (−45 °C), 1-Chlornaphthalin (−17 °C); Ester wie Essigsäuremethylester (−98 °C), Essigsäureäthylester (−83,6 °C); Nitromethan (−29,2 °C); aromatische Kohlenwasserstoffe wie o-Xylol (−27,9 °C), m-Xylol (−49,3 °C), tert.-Butylbenzol (−60,9 °C), Schwefelkohlenstoff (−112 °C); und entsprechende Gemische. Zweckmässig verwendet man das Lösungsmittel in einer Menge von 200 bis 10 000 Gewichtsprozent, vorzugsweise von 300 bis 2 000 Gewichtsprozent, bezogen auf Äthylisocyanat.

Die Reaktion wird unter Bestrahlung mit Licht, in der Regel UV-Licht, zweckmässig von Wellenlängen zwischen 200 und 700, vorzugsweise zwischen 200 und 400 Nanometer durchgeführt. Als UV-Lichtquelle können beliebige Quellen mit starker Strahlung im Ultraviolettbereich, z.B. Kohlebogen-, Quecksilberdampf-, Fluoreszenz-, Argonglühlampen oder Xenonlampen, verwendet werden. Die Lichtquellen sollen vorteilhaft, gegebenenfalls mit Hilfe von Reflektoren, Flächenlicht liefern. In der Regel kommen Belichtungszeiten von 30 bis 360, vorzugsweise von 120 bis 300 Minuten, in Frage. Bezüglich der Durchführung der Belichtung und der Lichtquellen wird auf Ullmanns Encyklopädie der technischen Chemie, Band I, Seiten 762 ff, verwiesen. Zweckmässig bestrahlt man mit 0,2 bis 10 000 Wattstunden, vorzugsweise 20 bis 1 000 Wattstunden, insbesondere 100 bis 500 Wattstunden Licht je Kilogramm Ausgangsstoff. Man kann die Lichtquelle auch in den Reaktionsraum, z.B. in Gestalt einer Tauchlampe, einführen. Das Reaktionsgemisch kann vorteilhaft in einem kontinuierlich arbeitenden Durchflussreaktor oder bei diskontinuierlicher Verfahrensweise in einzelnen Ansätzen bestrahlt werden. Geeignet ist auch die Durchmischung des Reaktorinhalts durch intensives Umwälzen z.B. eines Teilstroms durch einen externen Kreislauf, der zugleich für die Temperierung des Reaktionsgutes ausgenutzt werden kann. Das Verfahren lässt sich kontinuierlich durchführen, wobei die Photoreaktoren in der Reihe oder parallel betrieben werden können. Besonders vorteilhaft sind Fallfilm-Photoreaktoren, die es gestatten, die Ausgangsverbindungen in einer nichtstationären Flüssigkeitsschicht geringer Dicke als Gas-Flüssig-Reaktion unter Bestrahlung umzusetzen; es wird in diesem Zusammenhang auf Angew. Chem., Band 86 (1974), Seiten 706–707, und Tetrahedron Letters (1976), Seiten 4 057–4 060 verwiesen.

Die Reaktion kann wie folgt durchgeführt werden: Ein Gemisch von Chlor, Ausgangsstoff und Lösungsmittel wird unter guter Durchmischung während der vorgenannten Reaktionszeit bei der Reaktionstemperatur chloriert und dabei mit einer UV-Lichtquelle belichtet. Gegebenenfalls kann man das Reaktionsgemisch am Ende der Reaktion ohne zusätzliche Chlorzufuhr noch mit UV-Licht belichten (Nachreaktion), z.B. in den letzten 10 bis 90 Minuten der Reaktion, oder ohne Belichtung noch zusätzlich Chlor zuführen (Nachreaktion), z.B. in den letzten 10 bis 60 Minuten der Reaktion. Solche Nachreaktionen, denen entweder die Belichtung oder die Chlorzufuhr fehlen, betragen vorteilhaft von 0 bis zum vierten Teil der gesamten Reaktionszeit.

Bei Chlorierung ohne Lösungsmittel wird zweckmässig lediglich ein teilweiser Umsatz in dem Masse angestrebt, dass entstehendes 1-Monochloräthylcarbamidsäurechlorid unter den jeweiligen Konzentrations- und Temperaturbedingungen nicht als Feststoff ausfällt. Vorzugsweise beträgt der Umsatz 15 bis 50 Prozent.

Zweckmässig sorgt man durch Umwälzung und Bestrahlung in einer nichtstationären Schicht geringer Dicke unter fortwährender Kühlung für eine sofortige Umsetzung des in das Reaktionsgemisch eingeleiteten Chlors. Dann wird der Endstoff in üblicher Weise, z.B. durch Kristallisation und Filtration, abgetrennt und kann weiter umgesetzt werden. Es ist jedoch auch möglich, das rohe Reaktionsgemisch selbst weiter umzusetzen, ohne den erfindungsgemässen Endstoff zu isolieren.

Zum Erzielen einer hohen Ausbeute und Reinheit an 1-Chloräthylcarbamidsäurechlorid ist es zweckmässig, in der Zeiteinheit nur so viel Chlor zuzugeben wie in der gleichen Zeiteinheit mit dem Äthylisocyanat sich umsetzt. Der Überschuss sollte 0,1 Mol, vorzugsweise 0,05 Mol freies Chlor je Mol Äthylisocyanat im Reaktionsgemisch nicht übersteigen.

In einer bevorzugten Ausführungsform wird das Reaktionsgemisch nicht fraktioniert destilliert, sondern zunächst das Lösungsmittel abgetrennt, zweckmässig durch Destillation im Vakuum bzw. unter 30 °C, und der Rückstand umkristallisiert, z.B. aus Dichlormethan, Trichlormethan, Tetrachlormethan, n-Pentan, n-Hexan, Diäthyläther, Methyl-tert.-butyläther, 1,2-Dichloräthan, 1,1,1-Trichloräthan, 1,1,2-Trichloräthan, wobei der Endstoff in reiner Form anfällt. Ebenfalls ist eine bevorzugte Ausführungsform, das Reaktionsgemisch abzukühlen und den ausfallenden Endstoff abzufiltrieren. Es ist zweckmässig, bei der Aufarbeitung des Reaktionsgemisches Temperaturen von +30 °C, vorzugsweise +10 °C, bevorzugt −10 °C nicht zu überschreiten.

Das erfindungsgemäss herstellbare 1-Chloräthylcarbamidsäurechlorid ist ein wichtiger Ausgangsstoff für die Herstellung von 1-Chloräthylisocyanat (Ia) und Vinylisocyanat (Ib). So kann man ihn zum Beispiel bei einer Temperatur von −10 bis +150 °C in einer Reaktionszeit von 0,5 bis 6 Stunden in Gegenwart von α-Pinen, vorteilhaft 2 bis 20 Mol α-Pinen je Mol Endstoff, zu einem Gemisch von 1-Chloräthylisocyanat Ia und Vinylisocyanat Ib umsetzen, das als Gemisch weiterverarbeitet werden kann; aus dem Reaktionsgemisch können auch die Endstoffe Ia und Ib, zweckmässig direkt nach der Reaktion durch Erhöhung der Temperatur und fraktionierte Destillation, abgetrennt werden.

Ebenfalls kann man das 1-Monochloräthylcarbamidsäurechlorid der Formel

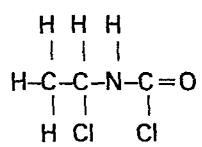

II,

a1) mit einem halogenfreien Isocyanat der Formel

$$R^1{-}N{=}C{=}O \qquad III,$$

worin $R^1$ einen Alkylrest, Cycloalkylrest, Arylrest, Aralkylrest oder Alkylarylrest bedeutet, und/oder

a2) mit einem Diisocyanat der Formel

$$O{=}C{=}N{-}R^2{-}N{=}C{=}O \qquad IV,$$

worin $R^2$ einen Alkylenrest, Cycloalkylenrest, Arylenrest, Alkylarylenrest oder Arylalkylenrest bezeichnet, umsetzen oder

a3) mit Vinylisocyanat Ib zu einem Gemisch von 1-monochloriertem Äthylisocyanat der Formel

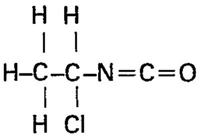

Ia

und Vinylisocyanat der Formel

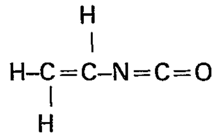

Ib

umsetzen.

Die Umsetzung kann für den Fall der Verwendung von 1-Chloräthylcarbamidsäurechlorid, Hexamethylendiisocyanat oder Vinylisocyanat durch die folgenden Formeln wiedergegeben werden:

a2) 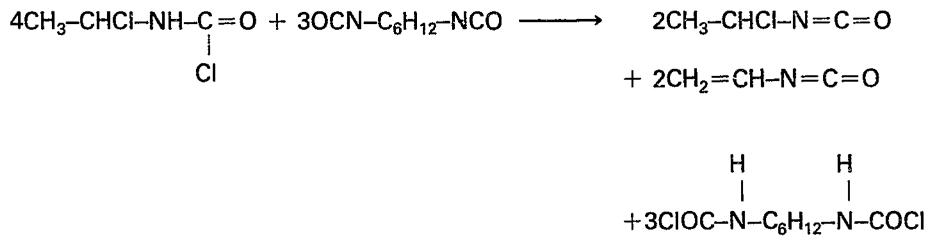

a3) 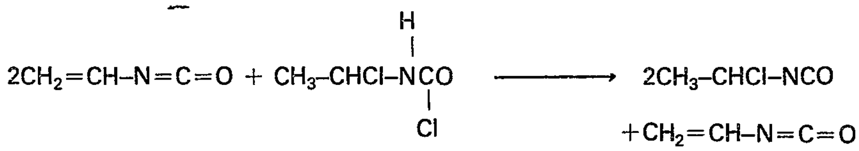

Man kann die Ausgangsstoffe II und III (Umsetzung a1) oder die Ausgangsstoffe II und IV (a2) oder die Stoffe Ib und II (a3) jeweils miteinander in stöchiometrischer Menge oder im Überschuss jeder Komponente zur anderen umsetzen, zweckmässig im Falle des Verfahrens a1) von 15 bis 25 Mol Stoff III je Mol Stoff II, im Falle des Verfahrens a2) von 3 bis 14 Mol Stoff IV je Mol Stoff II, im Falle des Verfahrens a3) von 0,5 bis 1 Mol Stoff II je Mol Stoff Ib. Man erhält im allgemeinen im Falle der erfindungsgemässen Umsetzung a1) Gemische von 0,05 bis 0,2 Mol Endstoff Ia je Mol Endstoff Ib, bei Verwendung vorgenannter zweckmässiger Mengen an Ausgangsstoffen von 0,05 bis 0,1 Mol Endstoff Ia je Mol Endstoff Ib, im Falle der Umsetzung a2) Gemische von 0,2 bis 0,7 Mol Endstoff Ia je Mol Endstoff Ib, bei Verwendung vorgenannter zweckmässiger Mengen an Ausgangsstoffen von 0,3 bis 0,6 Mol Endstoff Ia je Mol Endstoff Ib, im Falle der Umsetzung a3) Gemische von 2 bis 20 Mol Endstoff Ia je Mol Endstoff Ib, bei Verwendung vorgenannter zweckmässiger Mengen an Ausgangsstoffen von 7 bis 20 Mol Endstoff Ia je Mol Endstoff Ib. Je höher man die Konzentration an den Stoffen a1) III, a2) IV, a3) II im Vergleich zum anderen jeweiligen Ausgangsstoff wählt, desto grösser wird der Anteil an Endstoff Ib im Gemisch im Falle des Verfahrens a1) und entsprechend von Ib im Gemisch bei Verfahren a2), Ia im Gemisch bei Verfahren a3). Durch einen entsprechenden Versuch kann man somit leicht für jedes Verfahren ein gewünschtes Mengenverhältnis der Endstoffe Ia und Ib bzw. die wesentliche Bildung nur einer Komponente im Endstoffgemisch festlegen. Im Falle des Verfahrens a3) erhält man im wesentlichen nur den Endstoff Ia, im Falle des Verfahrens a1) und a2) im wesentlichen nur den Endstoff Ib.

Bevorzugte Ausgangsstoffe III, IV und demzufolge bevorzugte Endstoffe Ia und Ib sind solche, in deren Formeln der Rest $R^1$ einen Alkylrest mit 1 bis 12, insbesondere 1 bis 6 Kohlenstoffatomen, einen Cyclohexylrest, einen Aralkylrest oder Alkylarylrest mit 7 bis 12 Kohlenstoffatomen, oder einen Phenylrest bezeichnet, $R^2$ für einen Alkylenrest mit 1 bis 12 Kohlenstoffatomen, insbesondere 1 bis 16 Kohlenstoffatomen, einen Cyclohexylenrest, einen Aralkylenrest oder Alkylarylenrest mit 7 bis 12 Kohlenstoffatomen oder einen Phenylenrest steht. $R^1$ als Cycloalkylrest oder $R^2$ als Cycloalkylenrest können zweckmässig einen mono- oder bicyclischen, gegebenenfalls durch Alkylgruppen mit 1 bis 3 Kohlenstoffatomen substituierten Rest bezeichnen; im Falle eines bicyclischen Restes können die beiden Cycloalkylkerne anelliert oder direkt oder über eine Methylengruppe miteinander verbunden sein, die beiden Isocyanatogruppen im Falle der Ausgangsstoffe IV können 1) beide an einem Cycloalkylkern oder 2) an einem der beiden Cycloalkylkerne oder 3) jeweils nur eine Gruppe an einem der beiden Cycloalkylreste und die andere Isocyanatogruppe an dem anderen der beiden Cycloakylreste liegen oder 4) ein oder beide Isocyanatogruppen sind über Alkylengruppen mit 1 bis 3 Kohlenstoffatomen mit einem oder beiden Cycloalkylkernen verbunden. Der nach der Reaktion durch z.B. Destillation aus dem Gemisch abgetrennte Endstoff Ib kann bei dem Verfahren a3) erneut wieder für die Umsetzung zur Erzielung eines hohen Anteils an einem oder

anderen Endstoff verwendet werden. Gegebenenfalls kann man Ausgangsstoff II auch mit einem Gemisch der Ausgangsstoffe III und IV und Vinylisocyanat Ib umsetzen.

Geeignete Ausgangsstoffe III sind beispielsweise: Äthylisocyanat, Propylisocyanat, Isopropylisocyanat, Butylisocyanat, Isobutylisocyanat, sek.-Butylisocyanat, tert.-Butylisocyanat, Pentylisocyanat, 3-Methylbutylisocyanat, Hexylisocyanat, 2-Äthylhexylisocyanat, Cyclohexylisocyanat, Phenylisocyanat, Benzylisocyanat, 3-Methylphenylisocyanat, α-Naphthylisocyanat.

Als Ausgangsstoffe IV kommen z.B. in Frage: Hexamethylendiisocyanat, Toluylendiisocyanat, Bis-(3-methyl-4-isocyanato-cyclohexyl)-methan, 1,1,4,4-Tetramethylbutandiisocyanat(1,4), 1,1,6,6-Tetramethylhexandiisocyanat(1,6), 3-Isocyanatomethyl-3,5,5-trimethylcyclohexylisocyanat.

Vorteilhaft für die Auswahl der Ausgangsstoffe III und IV ist der bei einer erfindungsgemässen Umsetzung zu erwartende Siedepunkt des 1,2-ungesättigten Isocyanats Ib. Der Siedepunkt dieses Isocyanats sollte geringfügig, vorzugsweise jedoch deutlich niedriger, vorteilhaft 10 bis 100 °C niedriger, als der der Ausgangsstoffe III bis IV liegen. Ebenso sollte der Siedepunkt des 1-Chloräthylisocyanats möglichst tiefer, vorteilhaft 10 bis 50 °C tiefer, als der der Ausgangsstoffe II bis IV liegen. Andernfalls kann aber beim Abdestillieren eines Gemisches aus zwei oder drei Komponenten, z.B. der Isocyanate Ia, Ib und III, der Anteil an Endstoff Ia durch erneute Destillation des Ausgangsstoffs III fast vollkommen in das erwünschte Isocyanat Ib überführt werden. Bevorzugt für die Durchführung des erfindungsgemässen Verfahrens ist die sofortige und kontinuierliche Entfernung des gewünschten Gemisches oder einer der Endstoffe, vorzugsweise des Endstoffs Ib, aus dem Gleichgewicht mit Hilfe eines Inertgasstroms bei Normaldruck, besonders bei niedersiedenden Reaktionsprodukten oder durch Reaktionsführung im Vakuum oder auch durch Kombination von Inertgasstrom und die Arbeitsweise mit Vakuum, vorzugsweise jedoch durch einen Inertgasstrom, z.B. trockenen Stickstoff oder trockene Luft.

Ist in einem Fall der Verfahrensweise a1) oder a2) eine eindeutige Abtrennung des Isocyanats Ib bei der einmaligen Umsetzung von 1-Chloräthylcarbamidsäurechlorid II nicht zu erzielen, so kann zweckmässig das mit dem Trägergasstrom ausgetragene rohe Endstoffgemisch erneut mit Ausgangsstoff III oder IV zum gewünschten 1,2-ungesättigten Isocyanat umgesetzt werden. Der Reaktionsrückstand, der überwiegend aus dem Carbamidsäurechlorid der Ausgangsstoffe III oder IV besteht, kann in bekannter Weise zum Isocyanat regeneriert werden, z.B. durch thermische Chlorwasserstoffabspaltung und anschliessende Destillation des rohen Isocyanats, wobei der freiwerdende Chlorwasserstoff anderweitig zur Herstellung von z.B. wässriger Salzsäure Verwendung findet, d.h. der gesamte abgespaltene Halogenwasserstoff ist wirtschaftlich wieder nutzbar und recyclisierbar.

Je höher die Reaktionstemperatur und je länger die Reaktionszeit, desto höher ist der Anteil von Endstoff Ib im Endgemisch. Die Umsetzung wird in der Regel bei einer Temperatur von 0 bis 150 °C, im Falle der Herstellung von Gemischen mit mehr als 1,5, insbesondere oberhalb 1,5 bis 20 Mol Endstoff Ia je Mol Endstoff Ib, zweckmässig 0 bis 50 °C, im Falle der Herstellung von Gemischen mit 0,5 bis 1,5, insbesondere 0,9 bis 1,1 Mol Endstoff Ia je Mol Ib, zweckmässig 40 bis 70 °C, im Falle der Herstellung von Gemischen mit weniger als 0,5, insbesondere 0,05 bis unterhalb 0,5 Mol Endstoff Ia je Mol Endstoff Ib, zweckmässig 70 bis 120°, drucklos oder unter Druck, kontinuierlich oder diskontinuierlich durchgeführt. Vorteilhaft beginnt man die Umsetzung bei einer Temperatur von 0 bis 30 °C, erhöht langsam die Temperatur und beendet die Reaktion bei den vorgenannten, zweckmässigen Reaktionstemperaturen. Die Reaktionszeit beträgt im allgemeinen 0,1 bis 5 Stunden, vorteilhaft im Falle der Herstellung von Gemischen mit mehr als 1,5, insbesondere oberhalb 1,5 bis 20 Mol Endstoff Ia je Mol Endstoff Ib 2 bis 4 Stunden, im Falle der Herstellung von Gemischen mit 0,5 bis 1,5, insbesondere 0,9 bis 1,1 Mol Endstoff Ia je Mol Endstoff Ib 0,5 bis 3 Stunden, im Falle der Herstellung von Gemischen mit weniger als 0,5, insbesondere 0,05 bis unterhalb 0,5 Mol Endstoff Ia je Mol Endstoff Ib 1,5 bis 3 Stunden.

Bevorzugt setzt man in Abwesenheit von weiteren Lösungsmitteln um, jedoch können unter den Reaktionsbedingungen inerte Lösungsmittel verwendet werden. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Halogenkohlenwasserstoffe, insbesondere Chlorkohlenwasserstoffe, z.B. Tetrachloräthylen, 1,1,2,2- oder 1,1,1,2-Tetrachloräthan, Dichlorpropan, Methylenchlorid, Dichlorbutan, Isopropylbromid, n-Propylbromid, Butylbromid, Chloroform, Äthyljodid, Propyljodid, Tetrachlorkohlenstoff, 1,1,1- oder 1,1,2-Trichloräthan, Trichloräthylen; aliphatische oder cycloaliphatische Kohlenwasserstoffe, z.B. Heptan, Nonan, Benzinfraktionen innerhalb eines Siedepunktintervalls von 70 bis 190 °C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; und entsprechende Gemische. Zweckmässig verwendet man das Lösungsmittel in einer Mengevon 200 bis 10 000 Gewichtsprozent, vorzugsweise von 300 bis 2 000 Gewichtsprozent, bezogen auf Ausgangsstoff II.

Die Reaktion kann wie folgt durchgeführt werden: Ein Gemisch der Ausgangsstoffe wird bei der Reaktionstemperatur während vorgenannter Reaktionszeit umgesetzt. Aus dem Reaktionsgemisch werden dann die Endstoffe Ia und Ib, zweckmässig direkt während oder nach der Reaktion durch Erhöhung der Temperatur und fraktionierte Destillation, abgetrennt.

Das so herstellbare Vinylisocyanat Ib und 1-mono-Chloräthylisocyanat Ia sind wertvolle Ausgangsstoffe für die Herstellung von Schädlingsbekämpfungsmitteln, Farbstoffen, Pharmazeutika, Textilhydrophobierungsmitteln, Waschmitteln,

Kunststoffen, Bleichmitteln und Klebstoffen, da sie neben einer reaktiven Isocyanatgruppe noch eine aktivierte Doppelbindung bzw. ein aktiviertes α-C-Atom besitzen. Zudem ist Vinylisocyanat ein wichtiges Monomer, das in vielfältiger Weise zu Ketten- und Leiterpolymeren, wie z.B. strahlungshärtenden Lackharzen, umgesetzt werden kann (Chem. High Polymers (Tokyo) 13 (1956), Seite 390; J. Polymer Sc. 35 (1959), Seite 215, J. Org. Chem. 26 (1961), Seite 770; J. of Coatings Techn. 49 (1977), Seite 82). Die Stoffe Ia und Ib können zu Urethanen, z.B. für die Verwendung als Schaumstoffe oder hochmolekulare Überzüge mit hoher Flexibilität, oder zu Harnstoffen umgesetzt werden. Bezüglich der Verwendung wird auf vorgenannte Veröffentlichungen und Ullmanns Encyklopädie der technischen Chemie, Band 9, Seiten 11, 12, 404 sowie Band 17, Seite 204 (3. Auflage), hingewiesen.

Die in den folgenden Beispielen aufgeführten Teile bedeuten Gewichtsteile. Sie verhalten sich zu den Volumenteilen wie Kilogramm zu Liter.

Beispiel 1

Ein Gemisch aus 110 Teilen Äthylisocyanat und 440 Teilen Chlorbenzol wird in einem Fallfilm-Photoreaktor bei einer Temperatur von −3 °C mit einer Geschwindigkeit von 4 000 Volumenteile/Minute umgepumpt. Innerhalb von 4 Stunden werden unter Bestrahlung mit einer UV-Lampe 110 Teile Chlor eingeleitet. Es wird eine Stunde ohne weitere Gaseinleitung nachbestrahlt. Der Überschuss an Chlor während der gesamten Reaktion und Nachbestrahlungszeit beträgt weniger als 0,01 Mol je Mol unumgesetztes Äthylisocyanat. Man erhält 173 Teile (87,8% der Theorie) 1-Chloräthylcarbamidsäurechlorid bei einem Äthylisocyanat-Umsatz von 89,7% (gaschromatographisch geprüft).

Beispiel 2

Ein Gemisch aus 110 Teilen Äthylisocyanat und 440 Teilen Chlorbenzol wird in einem Fallfilm-Photoreaktor bei einer Temperatur von −10 °C mit einer Geschwindigkeit von 10 000 Volumenteile/Minute umgepumpt. Innerhalb von 2,5 Stunden werden unter Bestrahlung mit einer UV-Lampe 110 Teile Chlor eingeleitet. Der Überschuss an Chlor während der gesamten Reaktion und Nachbestrahlungszeit beträgt weniger als 0,05 Mol je Mol unumgesetztes Äthylisocyanat. Man erhält 151 Teile (89,4% der Theorie) 1-Chloräthylcarbamidsäurechlorid bei einem Äthylisocyanat-Umsatz von 84,6% (gaschromatographisch geprüft).

Beispiel 3

Ein Gemisch aus 110 Teilen Äthylisocyanat und 440 Teilen Chlorbenzol wird in einem Fallfilm-Photoreaktor bei einer Temperatur von −15 bis −20 °C mit einer Geschwindigkeit von 3 000 Volumenteile/Minute umgepumpt. Innerhalb von 5 Stunden werden unter Bestrahlung 80 Teile Chlor eingeleitet. Man bestrahlt ohne Gaseinleitung 45 Minuten nach und leitet anschliessend während 60 Minuten weitere 40 Teile Chlor unter Bestrahlung ein. Der Überschuss an Chlor während der gesamten Reaktion und Nachbestrahlungszeit beträgt weniger als 0,1 Mol je Mol unumgesetztes Äthylisocyanat. Es werden 173,5 Teile (98,9% der Theorie) 1-Chloräthylcarbamidsäurechlorid bei einem Äthylisocyanat-Umsatz von 87,8% erhalten (gaschromatographisch geprüft).

Beispiel 4

Ein Gemisch aus 110 Teilen Äthylisocyanat und 440 Teilen Chlorbenzol wird in einem Fallfilm-Photoreaktor bei einer Temperatur von −25 bis −30 °C mit einer Geschwindigkeit von 3 500 Volumenteile/Minute umgepumpt. Innerhalb von 4 Stunden werden unter Bestrahlung mit einer UV-Lampe 110 Teile Chlor eingeleitet. Es wird 30 Minuten ohne weitere Gaseinleitung nachbestrahlt. Der Überschuss an Chlor während der gesamten Reaktion und Nachbestrahlungszeit beträgt weniger als 0,01 Mol je Mol unumgesetztes Äthylisocyanat. Man erhält 172,5 Teile (97% der Theorie) 1-Chloräthylcarbamidsäurechlorid bei einem Äthylisocyanat-Umsatz von 89% (gaschromatographisch) geprüft).

Beispiel 5

Ein Gemisch aus 120 Teilen Äthylisocyanat und 720 Teilen Tetrachlorkohlenstoff wird in einem Fallfilm-Photoreaktor bei einer Temperatur von −15 bis −20 °C mit einer Geschwindigkeit von 4 000 Volumenteile/Minute umgepumpt. Innerhalb von 5 Stunden werden unter Bestrahlung mit einer UV-Lampe 120 Teile Chlor eingeleitet. Es wird eine Stunde ohne weitere Gaseinleitung nachbestrahlt. Der Überschuss an Chlor während der gesamten Reaktion und Nachbestrahlungszeit beträgt weniger als 0,02 Mol je Mol unumgesetztes Äthylisocyanat. Der gaschromatographisch geprüfte Äthylisocyanat-Umsatz beträgt 86,9%. Man zieht das Lösungsmittel im Vakuum weitgehend ab und erhält nach Filtration 172,3 Teile (82,7% der Theorie, bezogen auf umgesetztes $C_2H_5NCO$) 1-Chloräthylcarbamidsäurechlorid vom Fp 20 °C.

Beispiel 6 (Verwendung)

Ein Gemisch aus 142 Teilen 1-Chloräthylcarbamidsäurechlorid aus Beispiel 5, 92,5 Teilen tert.-Butylchlorid und 426 Teilen Hexamethyldiisocyanat wird bei einem Druck von 60 mbar 2,5 Stunden von 40 auf 78 °C erhitzt. Man erhält durch Destillation 89 Teile (84,3%) 1-Chloräthylisocyanat und 1,3 Teile (1,9%) Vinylisocyanat.

Beispiel 7

548 Teile Äthylisocyanat werden in einem Fallfilm-Photoreaktor bei einer Temperatur von −10 bis −15 °C mit einer Geschwindigkeit von 4 000 Volumenteile/Minute umgepumpt. Innerhalb von 4 Stunden werden 122 Teile Chlor unter UV-Bestrahlung eingeleitet. Der Überschuss an Chlor während der gesamten Reaktion und Nachbestrahlungszeit beträgt weniger als 0,01 Mol je Mol unumgesetztes Äthylisocyanat. Man erhält 215,7 Teile (88,3% der Theorie) 1-Chloräthylcarbamidsäurechlorid bei einem Teilumsatz von 19,7% (gaschromatographisch geprüft).

Beispiel 8 (Verwendung)

Das nach Beispiel 7 hergestellte Gemisch aus Äthylisocyanat und 1-Chloräthylcarbamidsäurechlorid wird unter Durchleiten von trockenem Stickstoff fraktionierend während 3,5 Stunden über eine Füllkörperkolonne destilliert. Man erhält 48,5 Teile (46,2% der Theorie, bezogen auf in Beispiel 7 chloriertes Äthylisocyanat) Vinylisocyanat vom Fp (1013 mbar) 38,5 °C und 331 Teile Äthylisocyanat vom Kp (1013 mbar) 60 °C.

Beispiel 9 (Verwendung)

Eine Lösung von 57 Teilen 1-Chloräthylcarbamidsäurechlorid in 100 Volumenteilen Hexamethylendiisocyanat wird langsam bei 100 °C während 0,5 Stunden unter die Oberfläche von 572 Teilen Hexamethylendiisocyanat zugegeben. Gleichzeitig bläst man Stickstoff durch die Lösung und kühlt das Abgas mittels einer Kühlfalle auf −100 °C. Man destilliert das Reaktionsgemisch fraktionierend. Man erhält 10,2 Teile (36,8% der Theorie) Vinylisocyanat vom Kp (1013 mbar) 38,5 °C und 6,8 Teile (16,1% der Theorie) 1-Chloräthylisocyanat vom Kp (101,3 mbar) 92 °C.

Beispiel 10 (Verwendung)

30 Teile 1-Chloräthylcarbamidsäurechlorid werden in 100 Volumenteilen 1-Chlornaphthalin gelöst und dem Gemisch während 0,3 Stunden bei 2 °C eine Lösung von 16 Teilen Vinylisocyanat in 20 Volumenteilen 1-Chlornaphthalin zugegeben. Man erhält 12,5 Teile (8% der Theorie) Vinylisocyanat und 43 Teile (92% der Theorie) 1-Chloräthylisocyanat (gaschromatographisch geprüft).

Beispiel 11 (Verwendung)

Man trägt bei 22 °C in 700 Volumenteile Isopropylisocyanat 57 Teile 1-Chloräthylcarbamidsäurechlorid ein, erwärmt unter Durchleiten eines schwachen Stickstoffstromes in 1,5 Stunden bis auf 70 °C und kondensiert das Reaktionsgemisch bei −70 °C in einer Kühlfalle. Man erhält 12 Teile (43% der Theorie) Vinylisocyanat vom Kp (1013 mbar) 38,5 °C und 0,85 Teile (2% der Theorie) Chloräthylisocyanat vom Kp (1013 mbar) 92 °C.

Beispiel 12 (Verwendung)

Man löst 57 Teile 1-Chloräthylcarbamidsäurechlorid in 520 Teilen Äthylisocyanat bei 22 °C und bläst trockenen Stickstoff durch die Lösung, wobei man eine Raschigringkolonne verwendet, um eine Vortrennung des 3 Komponentengemisches in der Kolonne (Äthyl-, Vinyl- und 1-Chloräthylisocyanat) zu erzielen. Man destilliert das Reaktionsgemisch fraktionierend während 3 Stunden. Man erhält 14 Teile (50,5% der Theorie) Vinylisocyanat vom Kp (1013 mbar) 38,5 °C und 466 Teile Äthylisocyanat vom Kp (1013 mbar) 60 °C.

Beispiel 13 (Verwendung)

563 Teile 1-Chloräthylcarbamidsäurechlorid werden in 2800 Volumenteilen Hexamethylendiisocyanat gelöst. Die Lösung von insgesamt 3100 Volumenteilen wird in 3 Teilen (500; 1500; 1100 Volumenteilen) mit unterschiedlicher Verweilzeit (150; 310; 225 Minuten) in einen Dünnfilmverdampfer gegeben, der bei Normaldruck mit einem Stickstoffgegenstrom betrieben wird. Die Manteltemperatur beträgt 73 bis 75 °C, die Übergangstemperatur 48 bis 54 °C. Das Reaktionsgemisch wird in einer Vorlage und zwei nachgeschalteten Kühlfallen aufgefangen und der Gehalt gaschromatographisch ermittelt. Man erhält 145,3 Teile (53% der Theorie) Vinylisocyanat vom Kp (1013 mbar) 38,5 °C und 73,8 Teile (17,7% der Theorie) 1-Chloräthylisocyanat vom Kp (1013 mbar) 92 °C.

Beispiel 14 (Verwendung)

Ein Gemisch aus 57 Teilen 1-Chloräthylcarbamidsäurechlorid, 156 Teilen Äthylisocyanat und 841 Teilen Hexamethylendiisocyanat wird unter Durchleiten eines Stickstoffstromes von 22 °C in 3 Stunden auf 90 °C erwärmt. Man erhält in der Destillationsvorlage ein Gemisch aus 11,8 Teilen (42,5% der Theorie) Vinylisocyanat vom Kp (1013 mbar) 38,5 °C, 10,1 Teilen (23,8% der Theorie) 1-Chloräthylisocyanat vom Kp (1013 mbar) 92 °C und 149 Teilen Äthylisocyanat.

Beispiel 15 (Verwendung)

Ein Gemisch aus 160 Teilen 1-Chloräthylencarbamidsäurechlorid und 537 Teilen α-Pinen wird während 2 Stunden unter Rühren von 24 auf 105 °C erwärmt und das Reaktionsgemisch fraktioniert destilliert. Man erhält insgesamt 27,6 Teile (23% der Theorie) 1-Chloräthylisocyanat vom Kp 92 °C (1013 mbar) und 34,6 Teile Vinylisocyanat vom Kp 38,5 °C (1013 mbar).

**Patentansprüche**

1. Verfahren zur Herstellung von 1-Monochloräthylcarbamidsäurechlorid durch Umsetzung von Isocyanaten oder Carbamidsäurehalogeniden mit Halogenierungsmitteln, dadurch gekennzeichnet, dass man Äthylisocyanat oder Äthylcarbamidsäurechlorid mit Chlor bei einer Temperatur von −78 bis 0 °C unter Belichtung umsetzt.

2. Verfahren nach Anspruch 1, dadurch gekennzeichnet, dass während der Reaktion ein Überschuss an freiem Chlor von höchstens 0,1 Mol je Mol Äthylisocyanat oder Äthylcarbamidsäurechlorid verwendet wird.

**Revendications**

1. Procédé de préparation du chlorure de monochloro-1 éthyl-carbamoyle par réaction d'isocyanates ou d'halogénures de carbamoyle avec des agents d'halogénation, caractérisé en ce que l'on fait réagir l'isocyanate d'éthyle ou le chlorure d'éthyl-carbamoyle avec le chlore à une température comprise entre −78 et 0 °C avec exposition à une source de lumière.

2. Procédé suivant la revendication 1, caractérisé en ce que, pendant la réaction, l'excès en chlore

libre ne dépasse pas 0,1 mole pour 1 mole d'isocyanate d'éthyle ou de chlorure d'éthyl-carbamoyle.

**Claims**

1. A process for the preparation of 1-monochloroethylcarbamyl chloride by reacting an isocyanate or carbamyl halide with a halogenating agent, wherein ethyl isocyanate or ethylcarbamyl chloride is reacted with chlorine at from −78 to 0 °C, under exposure to light.

2. A process as claimed in claim 1, wherein, during the reaction, an excess of free chlorine not exceeding 0.1 mole per mole of ethyl isocyanate or ethylcarbamyl chloride is used.